# EUROPEAN PATENT SPECIFICATION

(11) **EP 2 547 268 B1**
(45) Date of publication and mention of the grant of the patent: **06.01.2016**
(21) Application number: 11755575.5
(22) Date of filing: 18.03.2011
(51) Int. Cl.: A61B 17/16

(54) **DRILL BIT**
BOHRMEISSEL
MÈCHE

(30) Priority: 26.11.2010 AU 2010905238; 19.03.2010 AU 2010901172
(43) Date of publication of application: 23.01.2013
(73) Proprietor: CPL Holdings Pty Ltd, Blackalls Park, NSW 2283 (AU)
(72) Inventor: ELLIS, Liam Patrick, Blackalls Park, NSW 2283 (AU)
(74) Representative: Awapatent AB
(86) International application number: PCT/AU2011/000317
(87) International publication number: WO 2011/113115

(56) References cited:
- WO-A1-2009/141634
- US-A- 5 273 380
- US-A- 5 664 914
- US-A- 6 036 410
- US-A1- 2003 129 031
- US-B1- 6 235 035

## Description

### Technical Field

The present invention relates to the field of drill bits and in particular relates to, but is not limited to an orthopaedic drill bit.

### Background of the Invention

Drill bits are traditionally formed from a rod/shaft of high strength metallic material by grinding two or more helical gulleys, known as flutes, into the side wall of the rod extending from the operative front end of the rod towards the rear end, leaving a cylindrical shank at the rear end of the rod. The flutes are separated by lands that define the full diameter of the rod. To reduce the drag that would otherwise be experienced between the lands and the wall of the hole being drilled, the trailing region of each of the lands is ground in a second operation, providing a slightly reduced diameter over this portion of the drill bit known as a relief. This leaves only a leading portion, known as a margin of the land, defining the full diameter of the drill. The leading edge of the margin defines a secondary cutting edge with the trailing side wall of the adjacent flute, which is known as a cutting lip. During drilling operations, only the margin portion of the land engages the wall of the hole in the material being drilled, thereby significantly reducing drag acting on the drill bit and, accordingly, reducing the likelihood of the drill bit binding.

The cutting end part of the drill bit is formed by grinding the end region of the rod to provide a generally conical end part, known as a point, with end or tip faces extending from each land towards either a chiselled edge, for designs with two flutes, or a sharp point tip for designs with three or more flutes. A primary cutting edge is defined by the junction between the leading edge of each of the tip faces and the adjacent trailing side wall of the adjacent flute. It is these primary cutting edges that cut material being drilled at the end of the drill hole. The shavings of swarf cut from the material pass along the flutes towards the rear of the drill bit, thereby creating room for more material to be cut or shaved and passed into and along the flutes for ejection from the rear end of the flutes. Flute lengths are limited and once the flutes become full, material will stop flowing along the flute. The swarf material will then be directed back down towards the cutting face or radially outwards. This results in an increase of pressure within the flutes which may result in the drill bit ceasing to function properly. This pressure is dictated by the depth and width of the flutes.

Typical drill bit designs are also inefficient at passing the swarf material into the flutes, causing a build up of swarf material in the reliefs at the operating end of the drill bit, thus reducing its efficiency.

US-A-5 664 914 discloses a drill bit having a central axis and comprising a cylindrical holding section, a hexagonal prismal section and a top-truncated pyramid section having a plurality of faces. Several grooves extend along the hexagonal prismal and truncated pyramid section.

### Object of the Invention

It is an object of the present invention to substantially overcome or at least ameliorate at least one of the above disadvantages.

### Summary of the Invention

The present invention provides a drill bit having a central axis and comprising:
a tapered cutting end part terminating in a drill tip at one end of said drill bit and having at least one gulley extending toward said drill tip;
a shank extending from an opposing end of said drill bit ;
at least three flutes formed in said drill bit each said flute having a flute leading side wall and a flute trailing side wall; and
a land defined between each of said flutes and extending to said tapered cutting end part;
said cutting end part comprising at least three tip faces,
characterized in that each of said flutes helically extends from adjacent said shank into said cutting end part;
and in that each said tip face extends from one of said lands to said drill tip, each said tip face having a first tip face region extending from an end of said adjacent flute to said drill tip and a second tip face region extending from said first tip face region to the adjacent land, said second tip face regions being separated by said flutes;
and in that each said first tip face region has:
   a leading tip margin;
   a tip relief extending from said tip margin toward said first tip face region of an adjacent trailing tip face;
   one of said gulleys with a first end thereof communicating with an adjacent flute, each said tip margin defining a tertiary cutting edge with said gulley of a leading adjacent first tip face region; and
   a tip transition region blending said tip relief into said gulley;
   and in that in any cross section plane extending perpendicular to said central axis through said first tip face regions, said tip margin of each said first tip face region lies on a circle extending about said central axis and each said tip relief, each said tip transition region and each said gulley lies entirely within said circle.

For each said first tip face region, said tip margin may be defined by said tertiary cutting edge and thereby has a minimal width.

Also disclosed is a drill bit having a central axis and comprising:
a tapered cutting end part terminating in a drill tip at one end of said drill bit;
a shank extending from an opposing end of said drill bit;
a plurality of flutes formed in said drill bit and helically extending from adjacent said shank into said cutting end part, each said flute having a flute leading side wall and a flute trailing side wall; and
a land defined between each of said flutes and extending to said tapered cutting end part, each said land having:
   a leading land margin adjoining said flute trailing side wall of a said flute directly leading said land;
   a land relief extending from said margin toward a said flute directly trailing said land;
   a land transition region blending said land relief into said flute leading side wall of said flute directly trailing said land;
   wherein, in any cross sectional plane extending perpendicular to said central axis through said lands, said land margin of each said land lies on a circle extending about said central axis and each said land relief and each said land transition region lies entirely within said circle.

Typically, for each said land, said land margin defines a secondary cutting edge with said flute trailing side wall of said flute directly leading said land.

Typically, in each said cross-sectional plane for each said land, said relief is convexly curved.

Typically, in each said cross-sectional plane for each said land, said land relief is inclined with respect to said margin toward said central axis at a junction between said land relief and said land margin.

Preferably, in each said cross-sectional plane said land relief is inclined with respect to said land margin towards said central axis for each said land at a junction between said land relief and said land margin, at an angle of 5 to 15 degrees.

Alternatively, in each said cross-sectional plane for each said land, said land relief curves inwardly from said land margin towards said central axis.

Typically, in each said cross-sectional plane for each said land, said land transition region is curved and smoothly blends said land relief into said flute leading side wall of said adjacent flute directly trailing said land.

Typically, in each said cross-sectional plane for each said land, said land transition region has a radius of at least 0.2 times an overall diameter of said drill bit.

Typically, said drill bit has three said flutes.

Typically, said drill bit is an orthopaedic drill bit.

Also disclosed is a method of forming the above drill bit of the first aspect comprising:
grinding each said flute and adjacent said transition region and adjacent said relief in a single grinding operation with a single grinding wheel.

Also disclosed is a drill bit having a central axis and comprising:
a tapered cutting end part terminating in a drill tip at one end of said drill bit;
a shank extending from an opposing end of said drill bit;
at least three flutes formed in said drill bit and helically extending from adjacent said shank into said cutting end part, each said flute having a flute leading side wall and a flute trailing side wall; and
a land defined between each of said flutes and extending to said tapered cutting end part;
said cutting end part comprising at least three tip faces, each said tip face extending from one of said lands to said drill tip, each said tip face having a first tip face region extending from adjacent an end of said flutes to said drill tip and a second tip face rear region extending from said first tip face region to the adjacent said land, said second tip face regions being separated by said flutes;
each said second tip face region having:
   a leading face margin defining a primary cutting edge with said flute trailing side wall of a leading adjacent said flute;
   a face relief extending from adjacent said margin toward a trailing adjacent said flute; and
   a face transition region blending said face relief into said flute leading side wall of said trailing adjacent flute;
   wherein, in any cross sectional plane extending perpendicular to said central axis through said second tip face regions, said face margin of each said second tip face region lies on a circle extending about said central axis and each said face relief and each said face transition region lies entirely within said circle.

For each said second tip face region, the leading face margin may be defined by said primary cutting edge and thereby have a minimal width.

### Brief Description of the Drawings

A preferred embodiment of the present invention will now be described, by way of an example only, with reference to the accompanying drawings wherein:
Figure 1 is a perspective view of a drill bit;
Figure 2 is an enlarged perspective view of the cutting end part of the drill bit of Figure 1;
Figure 3 is a front elevation view of the drill bit of Figure 1;
Figures 3a through 3e are each cross-sectional views of the drill bit of Figure 1 taken at sections A-A to E-E of Figure 3 respectively;
Figure 4 is an end elevation view of the drill bit of Figure 1;
Figure 5 is a fragmentary front elevation view of the drill bit of Figure 1;
Figures 5h through 5k are each cross-sectional views of the drill bit of Figure 1 taken at sections H-H through K-K of Figure 5 respectively; and
Figure 6 is a fragmentary cross-sectional view of a grinding wheel for use in forming the drill bit of Figure 1; and
Figure 7 is a cross-sectional view, corresponding to Figure 3a, of a modified form of the drill bit of Figure 1.

### Detailed Description of the Preferred Embodiments

Referring to the accompanying drawings, a drill bit 1 has a tapered cutting end part 2 terminating in a drill tip 3 at a front, operative end of the drill bit 1, with a shank 4 extending from an opposing rear end of the drill bit 1. The shank 4 is configured to be received within the chuck of a drill in the usual way, and will typically be cylindrical although it may be hexagonal in cross-section or any other suitable shape. A plurality of flutes 5 are formed in the drill bit 1. In the embodiment depicted there are three flutes 5 that each extend from adjacent the shank 4 into the cutting end part 2. Each of the flutes 5 extends into the cutting end part 2 towards the drill tip 3, but finishes just short of the drill tip 3 as a result of the tapering of the cutting end part 2.

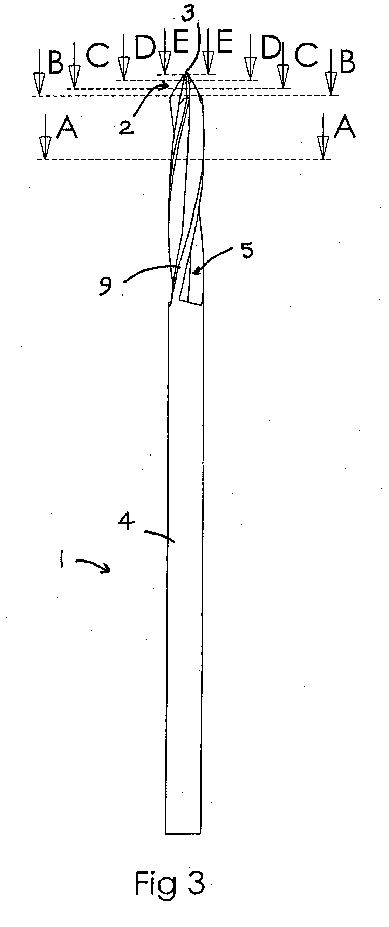

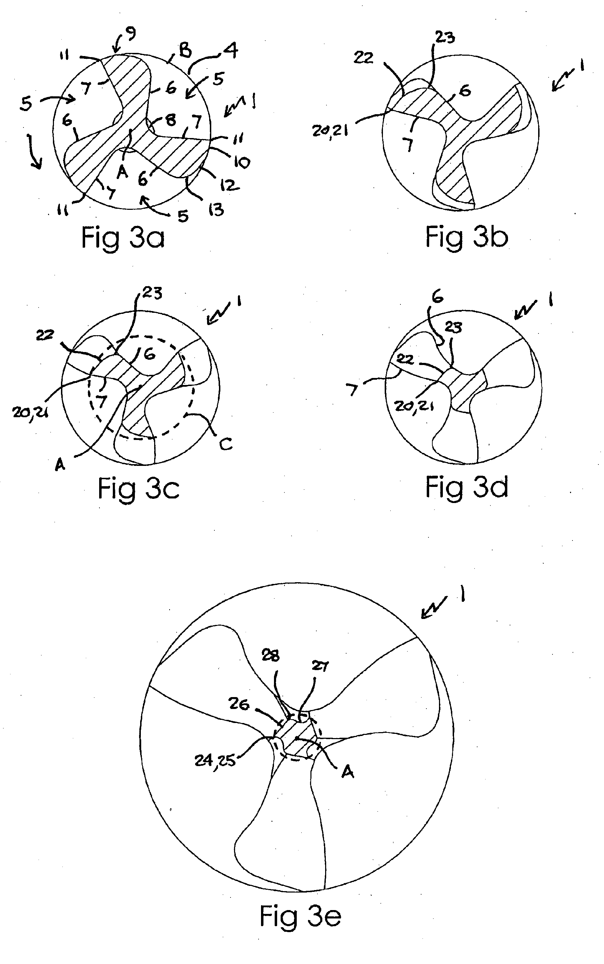

In the embodiment depicted, the drill bit 1 is configured to be rotated in a clockwise direction when viewed from the rear of the drill bit 1. Throughout this specification, various features of the drill bit will be referred to as "leading" or "trailing", with this terminology indicating features that lead or trail respectively as the drill bit rotates in the intended manner. Each of the flutes 5 has a flute leading side wall 6 (which faces against the intended direction of rotation) and a flute trailing side wall 7 (which faces in the intended direction of rotation). The flute leading side wall 6 is joined to the flute trailing side wall 7 by way of a flute base 8 located therebetween. As best depicted in the cross-sectional views of Figures 3a through 3d, the flute leading side wall 6, flute base 8 and flute trailing side wall 7 effectively form a smooth continuous surface. The flutes 5 are each formed with a helix angle, which may typically be about 13° or about 20° for the embodiment depicted, although the helix angle may be adjusted as desired for different applications. Typical helix angles will be between 8° and 45°, more typically between 8° and 20°. The helix of the flutes 5 is configured such that the rear end of each flute 5 trails the front end as the drill bit 1 rotates in the intended direction. The flute bases 8 have a slight taper of about 1 degree with respect to the central axis A of the drill bit, reducing the depth of the flutes 5 towards the shank 4.

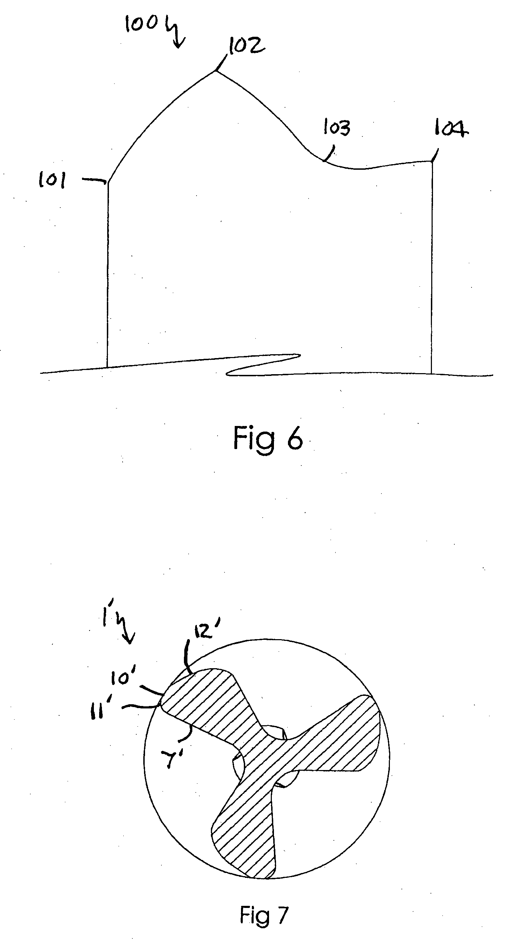

A land 9 is defined between each of the flutes 5. As best depicted in the cross-sectional view of Figure 3a, each land 9 has a leading land margin 10 which adjoins the adjacent flute trailing side wall 7 of the flute 5 directly leading the land 9, defining a secondary cutting edge 11. Each land 9 also has a land relief 12 which extends from the land margin 10 towards the adjacent flute leading side wall 6 of the flute 5 directly trailing the land 9. In a modified form of the drill bit 1', as depicted in Figure 7, the junction 11' between the land margin 10' and adjacent flute trailing side wall 7' may be convexly curved (or chamfered) such that, in use, it will perform only minimal or no cutting action.

A land transition region 13 blends the land relief 12 into the adjacent flute leading side wall 6, as best depicted in Figure 3a. This may be contrasted with a typical prior art drill bit wherein the land relief and flute leading side wall meet at a sharp edge. The transition region 13 will typically be curved so as to smoothly blend the land relief 12 into the flute leading side wall 6. The land transition region 13 will preferably have a radius, when measured in a cross-sectional plane perpendicular to the central axis A of the drill bit (such as the cross-sectional plane depicted in Figure 3a) of between 0.2 and 0.3 times the overall diameter of the drill bit 1. In the specific embodiment depicted, the drill bit 1 has a diameter of 4.5 mm, and the transition region has a radius of 1.15 mm. Rather than being curved, the land transition regions 13 could each be defined by one, or preferably two or more, chamfered surfaces.

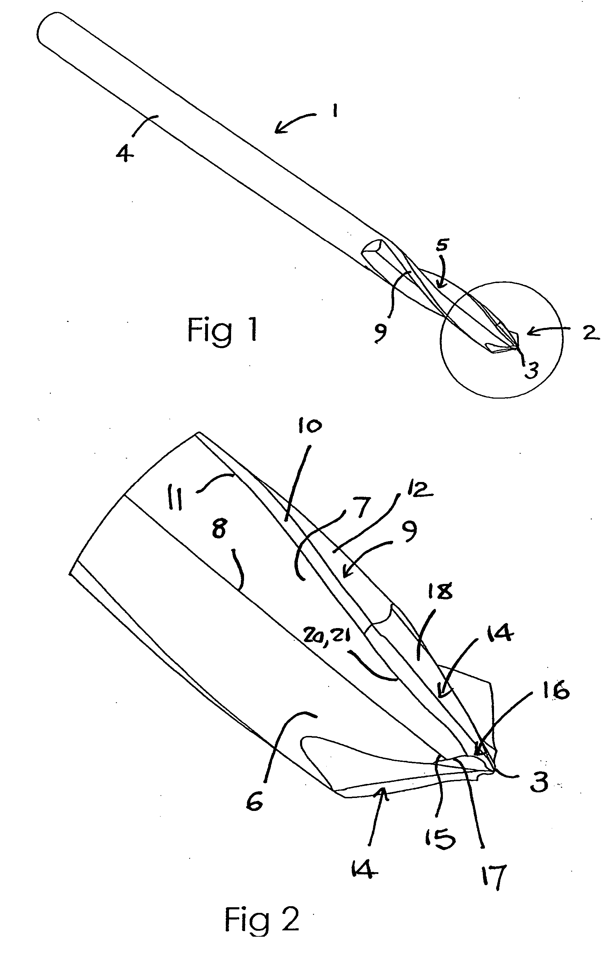

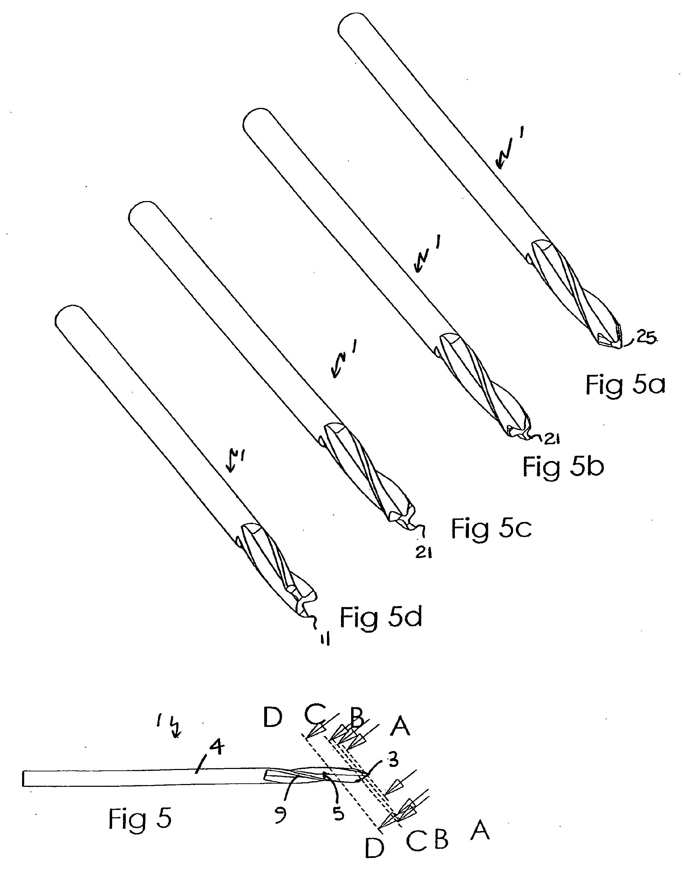

The land margin 10 constitutes a part cylindrical portion of the land 9 which is not ground away from the cylindrical shaft from which the drill bit 1 is formed. The land margin 10 has a width (measured in a cross-sectional plane) of about 0.2 mm in the embodiment depicted, however, it is envisaged that the land margin 10 may have a minimal width, effectively defined by the secondary cutting edge 11. The land margins 10 each lie on a circle B extending about the central axis A and having a diameter equal to the overall diameter of the drill bit (being equal to the diameter of the shank 4 in the embodiment depicted). The land relief 12 and land transition region 13 of each land are ground away from the cylindrical shaft from which the drill bit 1 is formed. Accordingly, at any cross-sectional plane extending perpendicular to the central axis A through the lands 9, each land relief 12 and land transition region 13 lies entirely within the circle B, as depicted in Figure 3a. The land relief 12 is convexly curved and is typically inclined with respect to the land margin 10 towards the central axis A at a junction between the land relief 12 and the land margin 10, defining an edge between the land margin 10 and the land relief 12. Alternatively, the land relief 12' may gradually curve inwardly from the land margin 10' towards the central axis A without leaving any definite edge therebetween, as per the modified form of drill bit 1' depicted in Figure 7. In the embodiment depicted in Figures 1 to 5, the land relief 12 is inclined with respect to the land margin 10, when measured in a cross-sectional plane, by about 11° degrees at the junction therebetween. Angles of inclination of 5 to 15 degrees would be typical. As such, the land relief 12 provides a greater area between its surface and the circle B than typical prior art designs, which are generally part cylindrical with a diameter only slightly less than the overall diameter of the drill bit.

The land relief 12 provides a clearance between the bulk of the land 9 and the wall of the hole being drilled, thereby reducing drill bit drag in the usual manner. As stated above, this clearance is generally greater than that provided with typical prior art designs, particularly towards the land transition region 13. This increased clearance, together with the additional clearance provided by the land transition region 13, provides an additional, secondary flow path for swarf to pass along the drill bit 1 as the flutes 5 begin to fill and increase pressure. The land transition region 13 also provides an opportunity for swarf material that is not immediately passed into the flutes 5, but has travelled into the land relief 12, to flow into the flutes 5. The land transition region 13 is believed to create a region of reduced pressure which actively draws swarf from the land relief 12 into the adjacent flute 5. Efficiency of the drill bit 1 is thus not affected by a build-up of swarf material caught within the land relief region 12.

Each of the flutes 5 and adjacent land transition region 13 and land relief 12 is formed by grinding the shaft from which the drill bit 1 is formed in a single grinding operation with a single shaped grinding wheel. This can be compared with a typical prior art process wherein the flute and relief are formed separately, with separate grinding operations utilising two separate grinding wheels. Accordingly, production of the drill bit 1 can be significantly more efficient than the prior art process. The profile of the outer operative portion of a grinding wheel 100 suitable for forming a flute 5 and adjacent land transition region 13 and land relief 12 in one operation is depicted in Figure 6. The profile effectively has four points 101-104 corresponding to specific points of the drill bit. The first point 101, at the left edge of the profile depicted, corresponds to the secondary cutting edge 11. The second point 102, forming a peak of the profile, corresponds to the flute base 8. The third point, forming a base of the profile, corresponds to the centre of the land transition region 13. The fourth point 104, at the right edge of the profile depicted, corresponds to the junction between the land relief 12 and the land margin 10.

The drill bit will typically be formed of stainless steel when configured for use as an orthopaedic drill bit, but other suitable high strength metallic materials may be utilised as desired.

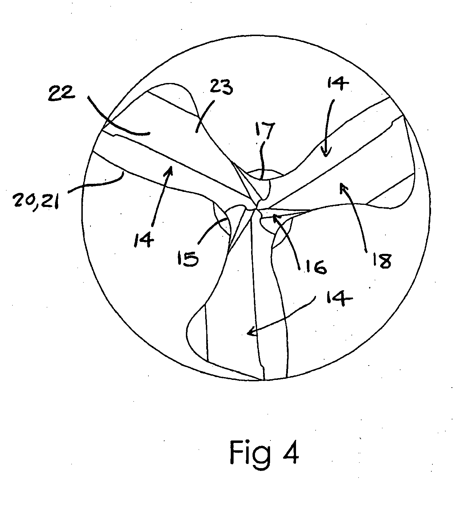

Referring specifically to Figures 2 and 4, the tapered cutting end part 2 of the drill bit 1 comprises three tip faces 14, one corresponding to each of the lands 9. Each tip face 14 extends from the corresponding land 9 to the drill tip 3 and effectively constitutes a tapered end of the corresponding land 9. The tip faces 14 define an included drill point angle, which is about 60° in the embodiment depicted, although the drill point angle may be altered as desired to suit the material to be drilled. Drill point angles of between 50° and 80° would be typical, and more typically between 60° and 80°. The flutes 5 extend between the tip faces 14 until they terminate where the tapered cutting end part 2 is tapered down to the thickness of the central web 15 that separates the flute bases 8.

Each tip face 14 is separated into two regions. A first tip face region 16 extends from adjacent the end 17 of each of the flutes 5 to the drill tip point 3. This first tip face region 16 constitutes the solid forward end of the cutting end part 2 where each of the tip faces 14 meets without a flute 5 therebetween. The second tip face region 18 constitutes the region extending from the first tip face region 16 to the forward end of the adjacent land 9. The second tip face regions 18 are each separated by one of the flutes 5.

Each of the second tip face regions 18 has a similar configuration to that of each of the lands 9. This configuration is best appreciated from each of the cross-sectional views of Figures 3b through 3d, representing successive cross-sectional planes taken through the second tip face regions 18. Each second tip face region 18 has a leading face margin 20 which defines a primary cutting edge 21 with the adjacent flute trailing side wall 7 of the flute 5 directly leading the second tip face region 18. For embodiments where the secondary cutting edges 11 are convexly curved or chamfered, it is envisaged that an aft transition region of each primary cutting edge 21 that adjoins an adjacent secondary cutting edge 11 may also be convexly curved or chamfered.

A face relief 22 extends from the face margin 20 towards the adjacent flute leading side wall 6 of the flute 5 directly trailing the second tip face region 18. In the embodiment depicted, the face margin 20 is effectively represented by a thin line defined by the primary cutting edge 21, rather than a more substantial margin as is the case with the land margin 10, with the face relief 22 smoothly blending into the face margin 20. A face transition region 23 blends the face relief 22 into the flute leading side wall 6. This can again be contrasted with a typical prior art drill bit where the tip face typically meets the flute leading side wall at a sharp edge.

Referring to Figure 3c, the face margins 20 each lie on a circle C extending about the central axis A, and having a diameter that is reduced as compared to the overall diameter of the drill bit given the tapering of the tapered cutting end part 2. The face relief 22 and face transition region 23 are ground away from the basic tapered form of the cutting end part 2. Accordingly, at any cross-sectional plane extending perpendicular to the central axis A through the second tip face regions 18, each face relief 22 and face transition region 23 lies entirely within the circle C as depicted in Figure 3C.

In a similar manner to the land relief 12, the face relief 22 reduces drag on the drill bit 1. The configuration of the face transition region 23, blending the face relief 22 into the adjacent flute leading side wall 6 also enhances the flow of excess swarf, which is initially passed into an area adjacent the face relief 22 rather than directly into the flute 5, in a similar manner to that discussed above in relation to the land transition region 13.

Referring to Figures 2, 3e and 4, the first drill face region 16 also shares some aspects of the configuration of both the lands 9 and the second tip face regions 18. Each first tip face region 16 has a leading tip margin 24 defining a tertiary cutting edge 25 with the trailing edge of the adjacent first tip face region 16. As with the face margins 20, the tip margins 24 will typically be defined by a line constituting the tertiary cutting edge 25. The tip margins 24 may, however, have a broader width. A tip relief 26 extends from the tip margin 24 towards the adjacent trailing first tip face region 16. A gulley 27 is formed in each first tip face region 16 between the tip relief 26 and the tertiary cutting edge 25 defined by the tip margin 24 of the directly trailing first tip face region 16. A curved tip transition region 28 blends each tip relief 26 into the adjacent gulley 27. The tip transition regions 28 each represent a continuation of an adjacent face transition region 23. Each of the gulleys 27 communicates with the end of an adjacent flute 5 and extends towards the drill tip 3 although it is envisaged that, in most applications, the gulley will not extend all the way to the drill tip 3 as can be seen in Figures 2 and 4. This is largely due to limitations in creating the gulleys 27 in such a fine region at the drill tip 3. It is envisaged that each gulley 27 may effectively be formed as a smooth continuation of the adjacent flute 5, such that each tertiary cutting edge 25 constitutes a smooth extension of the adjacent primary cutting edge 21.

The tip margins 24 each lie on a circle D extending about the central axis A as depicted in Figure 3e. Each tip relief 26, tip transition region 28 and gulley 27 lies entirely within the circle D.

The tip reliefs 26 again function in a similar manner to both the land reliefs 12 and face reliefs 22, both reducing drag and providing space for the passage of swarf. The gulleys 27 each provide a flow path for swarf from adjacent the drill tip 3 for feeding the swarf into the flutes 5. This may be contrasted with typical prior art drill bits, where the solid end portion of the cutting end part is substantially pyramid shaped without provision of any flow path for the passage of swarf. The arrangement of the gulleys 27 adjacent the tertiary cutting edges 25 also provides for much more efficient and sharper tertiary cutting edges 25 than is the case for pyramid shaped end part designs.

A person skilled in the art will appreciate that various modifications to the drill bit described may be made without departing from the scope of the disclosure of the present specification.

## Claims

1. A drill bit (1) having a central axis (A) and comprising:
a tapered cutting end (2) part terminating in a drill tip (3) at one end of said drill bit (1) and having at least one gulley (27) extending toward said drill tip (3);
a shank (4) extending from an opposing end of said drill bit (1);
at least three flutes (5) formed in said drill bit (1), each said flute (5) having a flute leading side wall (6) and a flute trailing side wall (7); and
a land (9) defined between each of said flutes and (5) extending to said tapered cutting end part (2);
said cutting end part (2) comprising at least three tip faces (14);
**characterized in that** each of said flutes (5) helically extends from adjacent said shank (4) into said cutting end part (2);
and **in that** each said tip face (14) extends from one of said lands (9) to said drill tip (3), each said tip face (14) having a first tip face region (16) extending from adjacent an end of said adjacent flute (5) to said drill tip (3) and a second tip face region (18)extending from said first tip face region (16) to the adjacent land (9), said second tip face regions (18) being separated by said flutes (5); and **in that** each said first tip face region (16) has:
a leading tip margin (24);
a tip relief (26) extending from said tip margin (24) toward said first tip face region (16) of an adjacent trailing tip face (14);
one of said gulleys (27) with a first end thereof communicating with an adjacent flute (5), each said tip margin (24) defining a tertiary cutting edge (25) with said gulley (27) of a leading adjacent first tip face region (16); and
a tip transition region (28) blending said tip relief (26) into said gulley (27);
and **in that** in any cross section plane extending perpendicular to said central axis (A) through said first tip face regions (16), said tip margin (24) of each said first tip face region (16) lies on a circle extending about said central axis (A) and each said tip relief (26), each said tip transition region (28) and each said gulley (27) lies entirely within said circle.

2. The drill bit of claim 1, wherein, for each said first tip face region (16), said tip margin (24) is defined by said tertiary cutting edge (25) and thereby has a minimal width.

3. The drill bit of claim 1, wherein each said second tip face region (18) has:
a leading face margin (20) defining a primary cutting edge (21) with said flute trailing side wall (2) of a leading adjacent flute (5);
a face relief (22) extending from said adjacent face margin (20) toward a trailing adjacent flute (5); and
a face transition region (23) blending said face relief (22) into said flute leading side wall (6) of said trailing adjacent flute (5);
wherein, in any cross sectional plane extending perpendicular to said central axis (A) through said second tip face regions (18) said face margin (20) of each said second tip face region (18) lies on a circle extending about said central axis (A) and each said face relief (22) and each said face transition (23) region lies entirely within said circle.

## Patentansprüche

1. Bohrer (1) mit einer mittleren Achse (A) und umfassend:
einen sich verjüngenden Schneidendenteil (2), der in einer Bohrerspitze (3) an einem Ende des Bohrers (1) endet und mindestens eine Furche (27) hat, die sich zur Bohrerspitze (3) erstreckt,
einen Schaft (4), der sich von einem gegenüberliegenden Ende des Bohrers (1) erstreckt,
mindestens drei Nuten (5), die im Bohrer (1) ausgebildet sind, wobei jede Nut (5) eine vordere Nutenseitenwand (6) und eine hintere Nutenseitenwand (7) hat, und
einen Rücken (9), der zwischen jeder der Nuten (5) definiert ist und sich zu dem sich verjüngenden Schneidendenteil (2) erstreckt,
wobei der Schneidendenteil (2) mindestens drei Spitzenflächen (14) umfasst,
**dadurch gekennzeichnet, dass** sich jede der Nuten (5) spiralförmig aus der Nähe des Schafts (4) in den Schneidendenteil (2) erstreckt
und dass sich jede Spitzenfläche (14) von einem der Rücken (9) zu der Bohrerspitze (3) erstreckt, wobei jede Spitzenfläche (14) einen ersten Spitzenflächenbereich (16), der sich aus der Nähe eines Endes der benachbarten Nut (5) zu der Bohrerspitze (3) erstreckt, und einen zweiten Spitzenflächenbereich (18), der sich von dem ersten Spitzenflächenbereich (16) zu dem benachbarten Rücken (9) erstreckt, hat, wobei die zweiten Spitzenflächenbereiche (18) durch die Nuten (5) getrennt sind,
und dass jeder erste Spitzenflächenbereich (16) Folgendes hat:
einen vorderen Spitzenrand (24),
eine Spitzenmulde (26), die sich von dem Spitzenrand (24) zu dem ersten Spitzenflächenbereich (16) einer benachbarten hinteren Spitzenfläche (14) erstreckt,
wobei eine der Furchen (27) mit einem ersten Ende davon mit einer benachbarten Nut (5) in Verbindung steht, wobei jeder Spitzenrand (24) eine tertiäre Schneidkante (25) mit der Furche (27) eines vorderen benachbarten ersten Spitzenflächenbereichs (16) definiert, und
einen Spitzenübergangsbereich (28), in dem die Spitzenmulde (26) in die Furche (27) übergeht,
und dass der Spitzenrand (24) jedes ersten Spitzenflächenbereichs (16) in einer beliebigen Querschnittsebene, die sich senkrecht zu der mittleren Achse (A) durch die ersten Spitzenflächenbereiche (16) erstreckt, auf einem Kreis liegt, der sich um die mittlere Achse (A) erstreckt, und jede Spitzenmulde (26), jeder Spitzenübergangsbereich (28) und jede Furche (27) gänzlich in dem Kreis liegen.

2. Bohrer nach Anspruch 1, wobei der Spitzenrand (24) für jeden ersten Spitzenflächenbereich (16) durch die tertiäre Schneidkante (25) definiert ist und dadurch eine minimale Breite hat.

3. Bohrer nach Anspruch 1, wobei jeder zweite Spitzenflächenbereich (18) Folgendes hat:
einen vorderen Flächenrand (20), der eine primäre Schneidkante (21) mit der hinteren Nutenseitenwand (2) einer vorderen benachbarten Nut (5) definiert,
eine Flächenmulde (22), die sich von dem benachbarten Flächenrand (20) zu einer hinteren benachbarten Nut (5) erstreckt, und
einen Flächenübergangsbereich (23), in dem die Flächenmulde (22) in die vordere Nutenseitenwand (6) der hinteren benachbarten Nut (5) übergeht,
wobei der Flächenrand (20) jedes zweiten Spitzenflächenbereichs (18) in einer beliebigen Querschnittsebene, die sich senkrecht zu der mittleren Achse (A) durch die zweiten Spitzenflächenbereiche (18) erstreckt, auf einem Kreis liegt, der sich um die mittlere Achse (A) erstreckt, und jede Flächenmulde (22) und jeder Flächenübergangsbereich (23) gänzlich in dem Kreis liegen.

## Revendications

1. Foret (1) comprenant un axe central (A) et comprenant :
une partie d'extrémité de coupe effilée (2) se terminant par une pointe de foret (3) à une extrémité dudit foret (1) et présentant au moins une gorge (27) s'étendant vers ladite pointe de foret (3) ;
une tige (4) s'étendant depuis une extrémité opposée dudit foret (1) ;
au moins trois cannelures (5) formées dans ledit foret (1), chacune desdites cannelures (5) ayant une paroi latérale avant de cannelure (6) et une paroi latérale arrière de cannelure (7) ; et
un méplat (9) défini entre chacune desdites cannelures (5) et s'étendant jusqu'à ladite partie d'extrémité de coupe effilée (2) ;
ladite partie d'extrémité de coupe (2) comprenant au moins trois faces de pointe (14) ;
**caractérisé en ce que** chacune desdites cannelures (5) s'étend en hélice depuis une position adjacente à ladite tige (4) jusque dans ladite partie d'extrémité de coupe (2) ;
et **en ce que** chaque dite face de pointe (14) s'étend depuis l'un desdits méplats (9) jusqu'à ladite pointe de foret (3), chaque dite face de pointe (14) ayant une première région de face de pointe (16) s'étendant depuis une position adjacente à une extrémité de ladite cannelure adjacente (5) jusqu'à ladite pointe de foret (3) et une deuxième région de face de pointe (18) s'étendant depuis ladite première région de face de pointe (16) jusqu'au méplat adjacent (9), lesdites deuxièmes régions de face de pointe (18) étant séparées par lesdites cannelures (5) ;
et **en ce que** chaque dite première région de face de pointe (16) présente :
une marge de pointe avant (24) ;
un relief de pointe (26) s'étendant depuis ladite marge de pointe (24) vers ladite première région de face de pointe (16) d'une face de pointe arrière adjacente (14) ;
l'une desdites gorges (27), dont une première extrémité communique avec une cannelure adjacente (5), chaque dite marge de pointe (24) définissant un bord de coupe tertiaire (25) avec ladite gorge (27) d'une première région de face de pointe adjacente avant (16) ; et
une région de transition de pointe (28) prolongeant ledit relief de pointe (26) jusque dans ladite gorge (27) ;
et **en ce que** dans tout plan en section transversale s'étendant perpendiculairement audit axe central (A) à travers lesdites premières régions de face de pointe (16), ladite marge de pointe (24) de chaque dite première région de face de pointe (16) est située sur un cercle s'étendant autour dudit axe central (A) et de chaque dit relief de pointe (26), chaque dite région de transition de pointe (28) et chaque dite gorge (27) étant situées entièrement à l'intérieur dudit cercle.

2. Foret selon la revendication 1, dans lequel, pour chaque dite première région de face de pointe (16), ladite marge de pointe (24) est définie par ledit bord de coupe tertiaire (25) et présente ainsi une largeur minimale.

3. Foret selon la revendication 1, dans lequel chaque dite deuxième région de face de pointe (18) présente :
une marge de face avant (20) définissant un bord de coupe primaire (21) avec ladite paroi latérale arrière de cannelure (2) d'une cannelure adjacente avant (5) ;
un relief de face (22) s'étendant depuis ladite marge de face adjacente (20) vers une cannelure adjacente arrière (5) ; et
une région de transition de face (23) prolongeant ledit relief de face (22) jusque dans ladite paroi latérale avant de cannelure (6) de ladite cannelure adjacente arrière (5) ; ;
ladite marge de face (20) de chaque dite deuxième région de face de pointe (18), dans tout plan en section transversale s'étendant perpendiculairement audit axe central (A) à travers lesdites deuxièmes régions de face de pointe (18), étant située sur un cercle s'étendant autour dudit axe central (A) et de chaque dit relief de face (22) et chaque dite région de transition de face (23) étant située entièrement à l'intérieur dudit cercle.
